# EUROPEAN PATENT APPLICATION

(11) **EP 3 434 293 A1**
(43) Date of publication of application: **30.01.2019**
(21) Application number: 16836071.7
(22) Date of filing: 14.12.2016
(51) Int. Cl.: A61L 31/12

(54) **ADHESION PREVENTION MATERIAL**

(30) Priority: 29.02.2016 WO PCT/JP2016/055988
(71) Applicant: Kawasumi Laboratories, Inc., Saiki-shi, Oita 876-0121 (JP); Shiga University Of Medical Science, Otsu-shi, Shiga 520-2192 (JP)
(72) Inventor: MUKAI Tomokazu, Bungo-Ono-shi Oita 879-7153 (JP); SEI Mariko, Bungo-Ono-shi Oita 879-7153 (JP); TANI Tohru, Otsu-shi Shiga 520-2192 (JP); KAMITANI Sumihiro, Otsu-shi Shiga 520-2192 (JP)
(74) Representative: Epping - Hermann - Fischer
(86) International application number: PCT/JP2016/087183
(87) International publication number: WO 2017/149897

(57) **Abstract**

PROBLEM TO BE SOLVED: To provide an anti-adhesion material capable of producing an excellent anti-adhesion performance without impairing degradability, handleability, and contact properties in actual use.

SOLUTION: An anti-adhesion material is provided that contains an ascorbic acid or anascorbic acid derivative as an antioxidant in a solid or semisolid base body made of a water-soluble polymer and a poly(aliphatic ester). Since the anti-adhesion material has the base body acting as a physical barrier inserted between the surfaces of a wound and the healing on the wound surfaces is promoted by the ascorbic acid or the derivative thereof being sustained-released, the anti-adhesion performance is extremely efficiently produced in a synergistic manner.

## Description

### TECHNICAL FIELD

The present invention relates to an anti-adhesion material. The present invention particularly relates to an anti-adhesion material comprising a solid or semisolid base body made of a water-soluble polymer and an aliphatic ester as well as an ascorbic acid or a derivative thereof as an antioxidant contained in the base body.

### BACKGROUND ART

The present patent applicant disclosed an invention of a novel anti-adhesion material in Patent Document 1 (Japanese Patent No. 5685297) which has an object to provide an anti-adhesion material excellent in (a) degradability when applied to a living tissue and (b) anti-adhesion performance in a living body and excellent in (c) handleability even when wetted and (d) contact properties when applied to a living tissue as compared to conventional materials.

The summary of the anti-adhesion material described in Patent Document 1 is that a water-soluble polymer is used for a base body layer and that a coating layer made up of an ultrathin film containing a biodegradable poly(aliphatic ester) is disposed on at least one or both surfaces of the base body layer (the ultrathin film results in the coating layer of 27 nm or more and less than 160 nm in optical thickness when measured by using a spectroscopic ellipsometer at a wavelength of 380 nm to 900 nm).

On the other hand, Patent Document 2 discloses an invention of a tissue anti-adhesion liquid containing trehalose as an active ingredient and an ascorbic acid derivative as an anti-oxidant.

### PRIOR ART DOCUMENTS

### PATENT DOCUMENTS

Patent Document 1: Japanese Patent No. 5685297 (Claims, Figs. 1 to 4)
Patent Document 2: Japanese Patent No. 4447640 (Claims, claims 1 to 3)

### SUMMARY OF THE INVENTION

### PROBLEMS TO BE SOLVED BY THE INVENTION

Although the invention described in Patent Document 1 is the invention providing an anti-adhesion material having a few excellent characteristics in a living body such as (a) degradability when applied to a living tissue, (b) handleability when wetted, and (d) contact properties when applied to a living tissue, it was recognized by the present inventors that as a result of further studies several problems for further improvement still remained in terms of ensuring a more sufficient (b) anti-adhesion performance, providing a more excellent anti-adhesion material, etc.

The tissue anti-adhesion liquid disclosed in Patent Document 2 is used in a spray system and is different in formulation from the film- or sheet-shaped anti-adhesion material described in Patent Document 1 and consequently has a different mechanism of performing an anti-adhesion function.

### MEANS FOR SOLVING PROBLEM

As a result of extensive studies for solving the problems described above, the present inventors conceived the following invention.
[1] The present invention provides an anti-adhesion material comprising an ascorbic acid or an ascorbic acid derivative contained as an antioxidant in a solid or semisolid base body made of a water-soluble polymer and a poly (aliphatic ester).
[2] The present invention provides the anti-adhesion material according to [1],wherein the solid base body has any one form selected from a film shape, a sheet shape, and a mesh shape, wherein
   the forms of the film shape, the sheet shape, and the mesh shape have a single layer structure substantially constituted by the base body or a laminated structure including a coating layer made of a poly(aliphatic ester) on a base body layer of the base body, and wherein
   the form of the mesh shape has a fibrous structure made up of a composite body acquired by forming a solid base body made of a water-soluble polymer and a poly (aliphatic ester) into a rod shape and has a weight of 0.8 g/m² to 830 g/m².
[3] The present invention provides the anti-adhesion material according to [1}, wherein the semisolid base body has a gel form including a colloidal form with a single layer structure or a laminated structure and has a viscosity of 100 Pa·s to 1,000,000 Pa·s at 37 °C.
[4] The present invention provides the anti-adhesion material according to [2], wherein the laminated structure including a coating layer on the base body layer of the base body includes respective coating layers on both surfaces of the base body layer and is formed in three layers, which are the coating layers on both surfaces and the base body layer.
[5] The present invention provides the anti-adhesion material according to [4], wherein the thickness of the coating layers is 50 nm or more and less than 7000 in optical thickness of the coating layers when measured by using a spectroscopic ellipsometer at a wavelength of 380 nm to 900 nm.
[6] The present invention provides the anti-adhesion material according to [1], wherein the ascorbic acid or the derivative thereof is contained at 0.1 (w/w) % or more to 20 (w/w) % or less relative to a weight (mass) of a base body material constituting the base body or the base body layer.
[7] The present invention provides the anti-adhesion material according to any of [1] to [6], wherein when the anti-adhesion material is dipped in bovine plasma, its pH becomes 7.5 to 6.0 after 24 hours.
[8] The present invention provides the anti-adhesion material according to any one of [2] to [7], wherein the anti-adhesion material includes as the coating layers a first coating layer disposed on one surface of the base body layer and a second coating layer disposed on the other surface of the base body layer, and wherein the first coating layer and the second coating layer are made of the same material.
[9] The present invention provides the anti-adhesion material according to any one of [2] to [7], wherein the anti-adhesion material includes as the coating layers a first coating layer disposed on one surface of the base body layer and a second coating layer disposed on the other surface of the base body layer, and wherein the first coating layer and the second coating layer are made of different materials.
[10] The present invention provides the anti-adhesion material according to any one of [1] to [9], wherein the water-soluble polymer is a polysaccharide, a protein, or a synthetic polymer.
[11] The present invention provides the anti-adhesion material according to any one of [1] to [10], wherein the water-soluble polymer (A) and the poly(aliphatic ester) (B) constituting the base body have a weight proportion ω (= A/B) of 1 to 99/99 to 1.
[12] The present invention provides an anti-adhesion material comprising an ascorbic acid or an ascorbic acid derivative contained, as an antioxidant, in a solid or semisolid base body substantially made of a water-soluble polymer, or the anti-adhesion material further comprising respective coating layers made of a poly(aliphatic ester) on both surfaces of a base body layer comprising the base body.
[13] The present invention provides the anti-adhesion material according to any one of [2] to [12], wherein the anti-adhesion material comprises a plant-derived antioxidant including carotenoids that are plant lipophilic pigments or polyphenols contained as the antioxidant of [1].

### ADVANTAGEOUS EFFECT OF THE INVENTION

The anti-adhesion material of the present invention is basically characterized by having a configuration comprising an ascorbic acid or an ascorbic acid derivative contained as an antioxidant in a solid or semisolid base body made of a water-soluble polymer and a poly(aliphatic ester).

In the anti-adhesion material of the present invention, because of this configuration,
<1> the base body made of a poly(aliphatic ester) and a water-soluble polymer acts as a physical barrier between the surfaces of wounds and the healing of the wound surfaces is promoted by the ascorbic acid or the derivative thereof which is being continuously released on the surfaces of the wounds, so that an anti-adhesion function is performed extremely efficiently in a synergistic manner.
<2> Since the shape of the base body is solid (a film shape, a sheet shape, a mesh shape) or semisolid (a gel form including a colloidal form) and, moreover, the ascorbic acid or the ascorbic acid derivative is contained in the base body or the base body layer in such a shape that function of the physical barrier can be ensured, thereby the effect of promoting the healing of the wound surfaces is achieved reliably on the surfaces of the wound. The ascorbic acid etc. contained in the base body or the base body layer are gradually eluted along with the water-soluble polymer which forms a viscous layer of solution on the base body surface and stays there (in the vicinity of the wound surfaces)to contribute to the healing promotion effect.
<3> Combining and using the ascorbic acid or the ascorbic acid derivative with the solid or semisolid base body advantageously enables continuous sustained release of the ascorbic acid to the wound surface. It is a general consideration that normally a wound requires at least 24 to 48 hours or, in some cases, one to two weeks for healing, and the ascorbic acid or the ascorbic acid derivative can continuously be sustained-released and stay on the wound surfaces over the required period.

With regard to the anti-adhesion material described in Patent Document 1, no consideration is given to the sustained release of the ascorbic acid or the ascorbic acid derivative because of the intention to accelerate degradability and absorbability of the base body layer and, even if the ascorbic acid or the ascorbic acid derivative is added as an anti-adhesion material component (wound healing promotion component), the sustained release of the ascorbic acid or the ascorbic acid derivative cannot be achieved and the sufficient anti-adhesion effect may not be produced for a sufficient period.

In particular, since the anti-adhesion material described in Patent Document 1 has a base body layer made of a water-soluble polymer, the base body layer is comparatively promptly dissolved into water and degraded/absorbed and, therefore, the anti-adhesion material has no room for considering the applicability of sustained release of the ascorbic acid from the base body layer.

In the case of the liquid form like the tissue anti-adhesion liquid described in Patent Document 2, similarly, considerations cannot be given to the continuous sustained release of drug to a wound surface and the mechanism of producing the anti-adhesion function is different from the present invention.

In this regard, as a result of extensive studies, the present inventors were able to confirm that the optimum sustained release can be ensured in the case of an appropriate or specific shape represented by a typical example described later.

### BRIEF DESCRIPTION OF DRAWINGS

Fig. 1 is a diagram for explaining an anti-adhesion material 1 according to a typical embodiment of the present invention, Fig. 1(a) is a perspective view of the anti-adhesion material 1, and Fig. 1(b) is a partially enlarged cross-sectional view thereof.
Fig. 2 is a graph of temporal evaluation of sustained release of an ascorbic acid added to a base body layer of the anti-adhesion material 1.

### MODES FOR CARRYING OUT THE INVENTION

### (Definition)

The following definition is given with respect to a "base body", a "base body layer", and a "coating layer" used in the description to articulately describe the anti-adhesion material of the present invention.

A "base body" is a main matrix member comprising a water-soluble polymer (A) and a poly(aliphatic ester) (B) for forming the anti-adhesion material. The anti-adhesion material is made by adding, and thus to be contained, an ascorbic acid or an ascorbic acid derivative (sometimes referred to as "ascorbic acid etc. ") to the "base body" . The base body gradually elutes or sustainedly releases contained ascorbic acid etc. into a living body while maintaining its shape for a certain period of time, when the thus-formed anti-adhesion material is placed in the biological body.

The base body specifically has a layered structure such as a single-layered or multi-layered structure to be preferably applied to an internal organ in a living system. The base body in case of a single-,or a mono-layered structure is called a "base body layer" . In the case of a multi-layered structure it is provided with an outer layer or outer layers (referred to sometimes a "coating layer" or "coating layers") covering one surface or both surfaces of the base body layer ("coating layer" is also referred to as "cover layer" in the present invention" .) . In most cases, the base body layer is relatively thick layer-structured and the cover layer is constructed as a thinner layer or layers.

The "coating layer" is a layer which mainly functions to control the rate or mode of the sustained release of the ascorbic acid etc. from the base body layer. The layer also functions as a reinforcing or "strength holding" layer of the base body layer, in order that its shape is kept substantially intact, when the anti-adhesion material is placed in a living body.

As described above, the "base body" is the matrix member to which an anti-oxidant such as ascorbic acid is added and from which the anti-oxidant is being control-released. In this regard, not only the "base body layer" but also "coating layer" is included in the definition of the "base body" (see Examples 5-6 below).

The main matrix member of the anti-adhesion material of the present invention comprises, by definition, a water-soluble polymer (A) and a poly(aliphatic ester) (B), wherein, in case of the single-layered structure, the layer essentially consists of a two-component composition (A/B) of a water-soluble polymer (A) and a poly(aliphatic ester) (B). While, in case of the multi-layered structure, specifically for a two-layered case consisting of a "base body layer" and a "coating layer", such a constitution is applicable, in that the "base body layer" is made to be composed essentially of a water-soluble polymer (A) and the "coating layer" is made to be composed essentially of a poly(aliphatic ester) (B) (see examples 1-4 below).

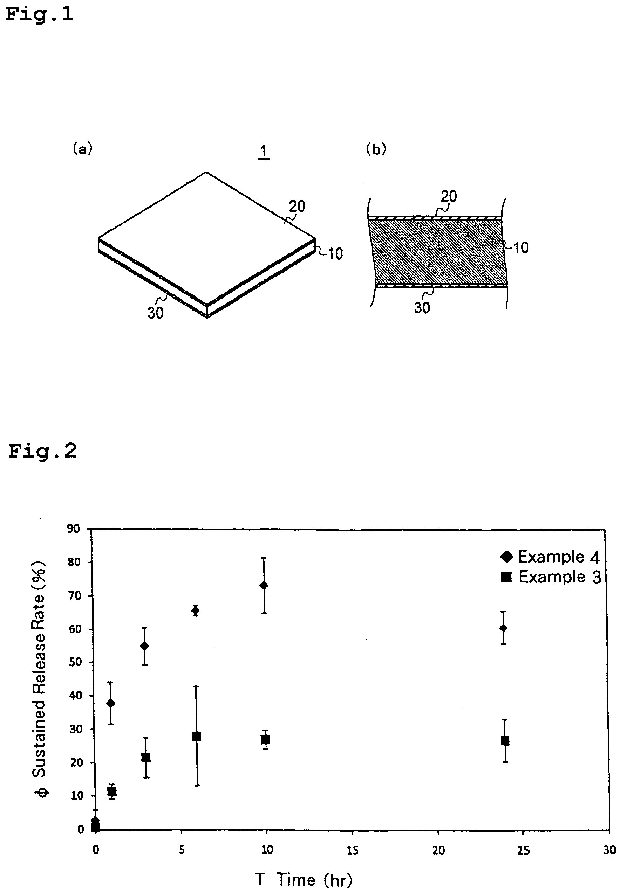

An anti-adhesion material of the present invention will now be described based on a typical embodiment shown in Fig. 1. The embodiment of the present invention is not limited to the embodiment shown in Fig. 1 and various embodiments can be achieved as long as the core concepts of the invention characterizing the present invention are included.

Generally describing the present invention, the anti-adhesion material of the present invention comprises an ascorbic acid or an ascorbic acid derivative (hereinafter sometimes referred to as "ascorbic acid etc.") contained in a so-called solid (or semisolid) base body made of a water-soluble polymer and a poly(aliphatic ester).

The anti-adhesion material, or specifically, the solid base body layer, has any one form selected from a film shape, a sheet shape, and a mesh shape, and a semisolid base body layer is in a gel form including a colloidal form.

If the solid base body has a shape of film or a sheet, the structure thereof is basically a base body layer comprising a single layer structure; however, if desired, the structure can be a laminated structure further including a coating layer on a base body layer as follows. In the case of such a laminated structure, the base body, most exemplary, is made up of a base body layer made of a water-soluble polymer and a coating layer made of a poly(aliphatic ester) formed thereon.

Although the film shape and the sheet shape are not definitely distinguished from each other in general, in the present invention, it is assumed that the film shape has a thickness less than 200 µm while the sheet shape has a thickness not less than 200 µm. In the case of the mesh shape, a fibrous structure is made up of a composite body which is acquired by forming a solid base body made of a water-soluble polymer and an aliphatic ester and formed into a rod shape and has a weight set within a range of 0.8 g/m² to 830 g/m².

If the solid base body is semisolid, the base body has a gel form including a colloidal form with a single layer structure or a laminated structure having a viscosity of 100 Pa·s to 1,000,000 Pa·s at 37 °C, and the base body with physical properties within this range functions as an effective physical barrier.

In any form, the base body is a so-called solid or semisolid base body made of a water-soluble polymer and an aliphatic ester containing an ascorbic acid etc., and the effect of the anti-adhesion material is based on a mechanism that an anti-adhesion function is effectively and reliably performed in a synergistic manner because wherein the base body acts as a physical barrier between surfaces of wounds and the sustained release of the ascorbic acid etc. to the barrier-protected wound surfaces promotes the healing of the wound surfaces.

### [Description of Typical Embodiment]

Fig. 1 is a diagram for explaining an anti-adhesion material 1 according to a typical embodiment of the present invention. The present invention is not limited to the typical embodiment described below. Although not shown, obviously, a coating layer can be formed on only one surface of a base body layer as shown in Fig. 2 of Patent Document 1, or coating layers can be formed on both surfaces of a base body layer as shown in Fig. 4 so as to achieve a form of a fibrous (mesh-shaped) structure made up of a composite body formed into a rod shape.

Fig. 1(a) is a perspective view of the anti-adhesion material 1, and Fig. 1(b) is a partially enlarged cross-sectional view of the anti-adhesion material 1. In Figs. 1(a) and 1(b), a layer thickness of a base body layer 10 and film thicknesses of a first coating layer 20 and a second coating layer 30 to the base body layer 10 are exaggerated to some extent in order to facilitate the understanding of the present invention.

As shown in Fig. 1, the anti-adhesion material 1 according to the typical embodiment includes the base body layer 10 formed into a film shape as well as the first coating layer 20 and a second coating layer 30 optionally disposed and located on one surface and the other surface, respectively, of the base body layer 10.

### (Basic Configuration of Base Body)

The anti-adhesion material of the present invention is characterized either by including the base body comprising a two-component resin composition (uniform resin mixture) made of a water-soluble polymer (A) and a biodegradable poly(aliphatic ester) (B) in the case of the single layer structure; or by including the solid or semisolid base body comprising a base body layer made of a water-soluble polymer (A) and a coating layer made of a biodegradable poly(aliphatic ester) (B) in the case of the laminated structure. In this regard, the anti-adhesion material is fundamentally different from the material disclosed in Patent Document 1 based on the base body layer made only of a water-soluble polymer (single component) .

Since the base body in the present invention has a single layer structure of a two-component composition made of a water-soluble polymer and a biodegradable poly (aliphatic ester) or a laminated structure, if the base body is placed in a living body, a poly(aliphatic ester) portion is not rapidly degraded (unlike a water-soluble polymer portion) and retains a layer-shaped (film-shaped or sheet-shaped) form for a significantly long period (a film-shape (or sheet-shape) retaining portion (shape-retaining portion)). On the other hand, the water-soluble polymer portion is mixed, or in contact, with the biodegradable poly(aliphatic ester) serving as the shape retaining portion and is therefore gradually eluted from a portion upon contacting a biological fluid on the surface of the base body (without comparatively promptly dissolving as in the case of a single component layer).

The (sustained-release) water-soluble polymer component gradually eluted from the base body in this way produces a healing-promotion activity on a wound surface.

### (Antioxidant/Ascorbic Acid etc.)

The anti-adhesion material of the present invention is characterized by comprising an ascorbic acid or an ascorbic acid derivative contained as an anti-oxidant in the base body either having the single layer structure made of the two-component composition configured as described above or the base body having the laminated structure.

According to the studies by the present inventors, even if the ascorbic acid etc. are contained in the base body with the intention to achieve sustained release from the base body, sufficient sustained release cannot be achieved in the case of the base body layer made only of a water-soluble polymer such as pullulan as disclosed in Patent Document 1, since the base body layer itself is dissolved in a comparatively short time as described above and stops its function as a matrix layer (form-retaining layer) . In contrast, in the case of the base body either having the single layer structure made of the two-component composition comprising a water-soluble polymer and a poly(aliphatic ester) or the base body having the laminated structure according to the present invention, the biodegradable poly(aliphatic ester) functions as a matrix (framework formation) and therefore the base body retains the form or shape without significant change for a long time.

On the other hand, in the present invention, the ascorbic acid etc. contained in the base body are eluted from the surface of the base body and, in the base body either having the single layer structure made of the two-component composition or the base body having the laminated structure, as described above, for example, the water-soluble polymer portion is mixed, or in contact, with the biodegradable poly(aliphatic ester) serving as the shape- retaining portion and is gradually eluted from the surface of the base body. It is considered that the eluted water-soluble polymer portion is eluted together with the ascorbic acid etc. (being contained in this portion) . Since the ascorbic acid etc. (healing component) are also gradually eluted along with the water-soluble polymer portion eluted from the base body in this way, it is believed that the highly-sustainable healing-promotion activity is produced.

### (Control of Sustained Release)

Since it is estimated that the sustained release mechanism of the ascorbic acid etc. contained in the base body in the present invention is as described above, the sustained release rate of the ascorbic acid etc. can be controlled in a wide range. In particular, when a proportion of a water-soluble polymer (A) is made larger in the base body either having the single layer structure comprising the two-component composition made of a water-soluble polymer (A) and poly (aliphatic ester) (B) or the laminated structure, an elution amount (elution rate) of the water-soluble polymer (A) becomes larger. (However, if the proportion of the water-soluble polymer is made excessively large, the biodegradable poly(aliphatic ester) (B) decreases and therefore the form-retaining capability deteriorates.)

On the other hand, if a proportion of poly (aliphatic ester) (B) is increased, the form of the base body such as a film shape is stably retained for a long time; however, conversely, the elution rates of the water-soluble polymer (A) and thus that of the ascorbic acid etc. are reduced. Considering the fact described above, the optimum sustained release can be implemented or achieved in accordance with its aimed or intended purpose.

From these points of view, in the present invention, desirably, a water-soluble polymer (A) and a poly(aliphatic ester) (B) constituting the base body have a weight proportion ω (= A/B) of 1 to 99/99 to 1, preferably, ω (= A/B) of 20 to 80/80 to 20, more preferably, ω (= A/B)of 30 to 70/70 to 30. If the proportion of the water-soluble polymer (A) is significantly smaller than this proportion, the elution rate thereof becomes too small and the sustained release rate of the contained ascorbic acid etc. becomes extremely small. On the other hand, if the amount of the poly(aliphatic ester) (B) is significantly larger than this proportion, the shape-retaining property is more improved; however, the sustained release of the water-soluble polymer (A) and the poly(aliphatic ester) (B) becomes too small.

The anti-adhesion material of the present invention has, most exemplary, in the case of the single layer structure, the base body made of a water-soluble polymer and a biodegradable poly (aliphatic ester), and in the case of the laminated structure it has the base body layer 10 made of a water-soluble polymer and the coating layer made of poly (aliphatic ester) as described above. The water-soluble polymer will first be described.

### (Water-Soluble Polymer)

For the water-soluble polymer, polysaccharides, proteins, or synthetic polymers can preferably be used.

Preferably usable polysaccharides include: for example, storage polysaccharides of animals and plants such as starch, amylose, amylopectin, glycogen, glucomannan, dextrin, glucan, and fructan; structural polysaccharides of animals and plants such as cellulose, pectin, and chitin; polysaccharides derived from seaweeds such as carrageenan and agarose; microbial polysaccharides such as pullulan; plant gum polysaccharides such as locust bean gum and guar gum; glycosaminoglycans such as heparin, hyaluronic acid, chondroitin sulfate, heparan sulfate, dermatan sulfate, and keratan sulfate; and derivatives of these polysaccharides.

Preferably usable proteins are, for example, gelatin, casein, and collagen.

Preferably usable synthetic polymers include: for example, polyvinyl alcohol, polyvinyl alcohol derivatives, polyacrylic acid-based water-soluble polymers, polyacrylamide, polyacrylamide derivatives, polyethylene oxide, polyethylene oxide derivatives, polyvinylpyrrolidone, polyvinylpyrrolidone derivatives, polyamide-based polymers, polyalkylene oxide-based polymers, polyether glycol-based polymers, and maleic anhydride copolymer-based polymers.

From the viewpoint of increasing the flexibility of the anti-adhesion material as a whole, in particular pullulan can be used most preferably among the exemplified water-soluble polymers.

### (Poly(Aliphatic Ester))

The poly(aliphatic ester) will be described.

Preferably usable poly(aliphatic esters) include: for example, poly(lactides); poly(glycolides); poly(lactide-ε-glycolides), poly(lactic acids); poly (glycolic acids),poly(lactic acid-ε-glycolic acids);polycaprolactones;polyesteramides;polyanhydrides;pol yorthoesters;polycyanoacrylates; polyether-esters; poly(dioxanones);poly(alkylene alkylates); copolymers of polyethylene glycol and polyorthoester, and copolymers thereof; and polymer alloys. Specifically, because of excellent biocompatibility and biodegradability, at least one of poly(lactic acids),poly(glycolic acids), polycaprolactones, and copolymers thereof is preferably used. In particular, preferably, a three-dimensional copolymer of lactic acid/glycolic acid/ε-caprolactone (LA/GA/ε-CLT) having a molecular weight of 20,000 to 300,000 are usable.

### (Base body Layer Thickness)

The layer thickness of the base body or the base body layer 10 (hereinafter sometimes simply referred to as the base body layer 10) is set to 1 µm to 5000 µm, for example.

In the case of the film-shaped anti-adhesion material, the layer thickness of the base body layer 10 is less than 200 µm, preferably 1 µm to 150 µm, more preferably 10 µm to 100 µm, most preferably 30 µm to 80 µm.

In the case of the sheet-shaped anti-adhesion material, the base body layer 10 is formed to have a layer thickness of 200 µm or more. The layer thickness is preferably 200 µm to 5000 µm, more preferably 300 µm to 3000 µm, further more preferably 500 µm to 2000 µm, most preferably 800 µm to 1000 µm. The thickness of the base body layer 10 can be measured by direct contact measurement using a caliper, a micro gauge, etc., or can be measured by employing suitable equipment such as an infrared film thickness meter, a capacitance type thickness meter, and a laser displacement sensor.

### (Ascorbic Acid, Ascorbic Acid Derivative)

In the present invention, an ascorbic acid or a derivative thereof is used as an anti-oxidative component that is the most preferable element for being sustained-released from the anti-adhesion material, and is contained in the base body or the base body layer (or, in some cases, in the coating layer) .

Ascorbic acid is one of organic compounds having a lactone structure and acting as a nutrient, vitamin C, and is represented by the following chemical formula I. According to (IUPAC nomenclature of chemistry), ascorbic acid is considered as a derivative of furan and is represented as (R)-3, 4-dihydroxy-5-((S)-1, 2-dihydroxyethyl) furan-2(5H)-one. Ascorbic acid is an optically active compound having the molecular weight of 176.13 g/mol, the melting point of 190 °C, and the boiling point of 553 °C. In the present invention, the L-form known as vitamin C, i.e., L-ascorbic acid is preferably used.

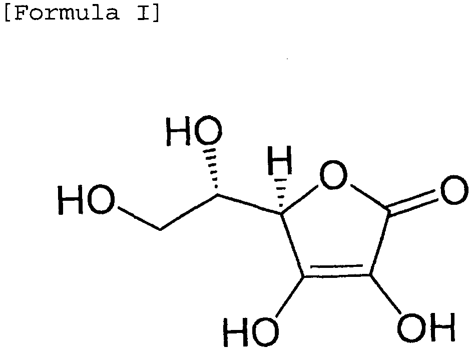

In the present invention, other usable ascorbic acid derivatives include: calcium ascorbate, sodium ascorbate, phosphoric acid-sodium L-ascorbate, phosphoric acid-magnesium L-ascorbate, ascorbic acid glucoside, ascorbylethyl, etc.

### (pH value)

The ascorbic acid or the ascorbic acid derivative is desirably added at lease at 0.1 (w/w) % or more to 20 (w/w) % or less, generally at 0.5 (w/w)% or more to 10 (w/w)% or less preferably 1.0 (w/w)% or more to 9.0 (w/w)%or less, more preferably 1.5 (w/w)% or more to 8.0 (w/w)%or less, relative to the weight (mass) of the whole anti-adhesion material. When the anti-adhesion material of the present invention has the ascorbic acid or the ascorbic acid derivative added to and contained in the base body or the base body layer at the above proportion and is immersed in 100×100 mm/30 ml bovine plasma, pH value thereof becomes 7.5 to 6.0 after 24 hours. This pH level is found to be most preferable for promotion of healing of a wound surface.

This means that when the anti-adhesion material of the present invention is disposed in a living body, pH value in the vicinity of the anti-adhesion material disposition portion does not change from an initial value and is maintained at a value of 7.5 to 6.0 optimum for healing, as long as the ascorbic acid etc. contained in the base body or the base body layer is sustained-released. Thus, the anti-adhesion material of the present invention enables varied and desirable sustained release in a wide range in accordance with an intended purpose and is capable of maintaining the vicinity of the wound surface at desired constant pH level for a long period.

Further describing this point, in the present invention, when the water-soluble polymer portion is gradually eluted from the surface of the base body, the ascorbic acid etc. are eluted together. Particularly, it is estimated that since the eluted water-soluble polymer such as pullulan forms a viscous solution, the solution stays at a position in the vicinity of the base body surface to cover it, while the ascorbic acid etc. being kept dissolved in the solution. It is supposed that the concentration of the ascorbic acid etc. in the viscous water-soluble polymer solution is substantially equal to the concentration in the base body Cm* (if the content of ascorbic acid etc. in the base body is Cm* = 8%, it is rational to consider that the concentration in the eluted water-soluble polymer solution is also approx. 8%).

Since a wound surface is stably covered with the viscous solution of pullulan etc. containing the ascorbic acid etc. in this way, it is considered that the anti-adhesion effect is promoted because the wound surface is protected by the synergistic effect of the ascorbic acid and pullulan etc.

The base body of the present invention may be formed by a film extrusion method or a sheet extrusion method from a predetermined resin composition of ascorbic acid etc., or may be formed by a flow casting method from a solution of the resin composition.

### (Other Antioxidants)

Although the ascorbic acid etc. are most effective in the present invention, the following anti-oxidants, for example, can be used if desired instead of the ascorbic acid etc. Specifically, plant-derived anti-oxidants (substances for SOD) can be used, including vitamin E; carotenoids that are plant lipophilic pigments such as α-carotene, β-carotene, γ-carotene, lycopene, and xanthophyll; and polyphenols contained in flowers, leaves, bark, stalk, etc. of plants such as flavonoid, catechin, tannin, anthocyanin, isoflavone, and quercetin.

### (Coating Layer)

In the present invention, basically, the function of the sustained release of the ascorbic acid etc. can be provided by the single layer structure substantially constituted of the base body made of a resin composition comprising a water-soluble polymer (A) and a poly (aliphatic ester) (B) as described above without a coating layer; however, the laminated structure can be implemented by further including a coating layer made of a poly (aliphatic ester) on the base body layer comprising the base body if desired. In the case of such a laminated structure, as described above, most exemplary, the base body is made up of the base body layer substantially made of a water-soluble polymer and the coating layer made of a poly(aliphatic ester) formed thereon. In this situation, the function of the coating layer comprising a poly(aliphatic ester) is basically to control the mode of sustained release of ascorbic acid added to the coating layer. In case where no coating layer were provided, ascorbic acid, along with water-soluble polymer, would elute and become exhausted in a short time, thus making it difficult to secure good and continued sustained release for sufficient long time.

Thus, in the case of the laminated structure, as shown in Fig. 1, the coating layer 20 (first coating layer) and the coating layer 30 (second coating layer) can be formed on one surface if desired and the other surface, respectively, of the base body layer 10.

### (Coating Layers Comprising Materials)

Both the first coating layer 20 and the second coating layer 30 acting as coating layers are preferably made of a biodegradable poly (aliphatic ester) . In other words, the same poly(aliphatic ester) as the constituent component of the base body is usable. For confirmation, examples will be listed again without sparing efforts of listing. Preferably usable poly(aliphatic esters) include: for example, poly(lactides); poly(glycolides); poly(lactide-ε-glycolides), poly(lactic acids); poly(glycolic acids),poly(lactic acid-co-glycolic acids);polycaprolactones;polyesteramides;polyanhydrides;pol yorthoesters;polycyanoacrylates;polyether-esters;poly(dioxa nones);poly(alkylene alkylates); copolymers of polyethylene glycol and polyorthoester, and copolymers thereof; and polymer alloys. Particularly, as in the case of constituting the base body layer, because of excellent biocompatibility and biodegradability, at least one of poly(lactic acids), poly(glycolic acids), polycaprolactones, and copolymers thereof is preferably used. A three-dimensional copolymer of lactic acid/glycolic acid/ε-caprolactone (LA/GA/ε-CLT) is particularly preferable. In the typical embodiment, the first coating layer 20 and the second coating layer 30 are preferably made of the same material out of these above-exemplified materials.

### (Coating layers Containing Ascorbic Acid)

Although the coating layers are basically or mainly made of these poly (aliphatic esters) as described above, in some cases ascorbic acid etc. may be further contained in the coating layers if desired. That is the case, as described later, where relatively thick coating layers are provided in an attempt to reinforce and retain the mechanical strength of the coating layer . Thus constituted, by containing the ascorbic acid etc. in the coating layers, the sustained release can reliably proceeds particularly even in an early stage when the anti-adhesion material is set in a wound portion. However, since the coating layers have a thickness Ld far thinner than the base body layer LD as described above, a total amount of the ascorbic acid etc. containable in the coating layers cannot be made so large, making it difficult to continue the sustained release for a sufficient long time (long period), and therefore, primarily, such constitution is preferably adopted for performing a subsidiary release to the main ascorbic acid etc. contained in the base body layer.

In the case of the single-layered structure of anti-adhesion material, ascorbic acid is added only to the base body layer, wherein its elution or sustained release occurs relatively promptly, since ascorbic acid belongs to water-soluble polysaccharide group. For this constitution, by providing or forming a thin coating layer, the dissolution rate of ascorbic acid can be appropriately so controlled that desired sustained release rate ϕ is preferably achieved, as exemplified by Examples 3-4 given later.

In the case of layered structure, when adopting thicker-than-usual coating layers, it is preferable to add ascorbic acid to both the base body layer and coating layer, as shown in the Examples 5-6. In case ascorbic acid is added to the coating layer in addition to the base body layer, sustained release rate (elution rate) ϕ from the coating layer is estimated to proceed in the following manner:

Specifically, in a case when the added ascorbic acid concentration (Cm) is kept constant, and the thickness of the coating layer (Ld) is varied, the thinner the coating layer, the greater the sustained release rate at an early stage. However, ascorbic acid in the coating layer is exhausted in a shorter time. In contrast, it is considered that when the thicker coating layer is adopted, the slower release rate is established, with the results that sustained release of it continues for a long period of time by virtue of the greater total amount of ascorbic acid contained in the coating layer.

On the other hand, consideration is given about the case when the thickness of the coating layer (Ld) is kept constant, and the added ascorbic acid concentration (Cm*) is varied, it is expected that the higher the concentration Cm*, the greater the ascorbic acid release rate at the initial stage, and the lower its concentration Cm*, the slower the elution rate.

As is described above, by making appropriate adjustments on coating layer thickness Ld and added ascorbic acid amount (concentration) Cm*, any variation in sustained release rate and its duration etc. can be achieved as desired, thus enabling to offer a number of therapeutic options for treatments.

The ascorbic acid content (concentration) Cm* in the coating layer is at lease 0.01 (w/w) % or more to 1.0 (w/w) % or less, preferably at 0.02 (w/w) % or more to 0.8 (w/w) % or less, more preferably at 0.1 (w/w)% or more to 0.5 (w/w)% or less relative to the weight (mass) of the coating layer.

### (Thickness of Coating Layers)

The function of the coating layers is, as described earlier, basically control of sustained release rate (elution or dissolution rate).

For example, as described later in Examples, by disposing a coating layer made of a biodegradable poly(aliphatic ester) and controlling the optical thickness thereof in a range of about 50 nm or mare and 800 nm or less, preferably 75 nm or more and less than 600 nm, the sustained release of ascorbic acid (from the base body layer) can be effectively varied. Moreover, although the weight proportion ω (= A/B) of the water-soluble polymer (A) and the poly(aliphatic ester) (B) constituting the base body layer can basically be varied to control the elution rate of the water-soluble polymer (A) (and thus the elution rate of the ascorbic acid etc.) in the present invention as described above, the sustained release can more finely be controlled by forming the coating layers and changing the thickness (Ld) thereof within the specific range as described above.

If an ascorbic acid or an ascorbic acid derivative is added to the base body layer 10, the coating layers (the first coating layer 20 and the second coating layer 30) are formed to be 50 nm or more and 800 nm or less, preferably 75 nm or more and less than 600 nm in optical thickness when measured by using a spectroscopic ellipsometer at a wavelength of 380 nm to 900 nm. Preferably, the coating layers are formed to be 75 nm or more and less than 500 nm.

Although when the coating layers (the first coating layer 20 and the second coating layer 30) have such a thin optical thickness (about 50-75 nm), in the present invention, as the good amount of ascorbic acid etc. being added to the base body layer 10, and thus a sufficient anti-adhesion performance can be secured.

Making the coating layers too thick (for example more than 800 nm) is not preferable because of a substantial reduction in degradability of the coating layers in a living body. And such thick coating layers come to prevent elution (diffusion) of ascorbic acid from the base body layer into the living body, making it also not desirable from the point of maintaining a steady sustained release . However, from the viewpoint of holding strength (holding of shape) of the base body layer (eventually of anti-adhesion material), the coating layers themselves are required to hold such sufficient strength or enough thickness so as not to be broken easily. In this regard, for example, as will be shown later by Examples 5-6, the coating layer should have a thickness basically of 800-7000 nm, preferably 900-5000 nm, more preferably 1000-3000 nm. Thus consideration of thickness of coating layers is required from the point of maintaining layers' strength.

Specifically, when conducting a study to apply an anti-adhesion material of the present invention to some bipedal animals such as monkey and human having an inner environment of higher inner pressure of abdominal cavity or frequent movement of inner organs such as stomach or intestine movement etc. , which possibly may cause disruption of film- or sheet-shaped membrane forming the base body (or coating layer) of the anti-adhesion material of the present invention.

As described above, too thick coating layers may generate concerns of causing a reduction in its degradability (or decrease in sustained release rate of ascorbic acid). However, when anti-adhesion material is to be applied to some bipedal animals such as monkey and human, sometimes priority must be given to maintain the mechanical strength of the coating layer, and in that case, the coating layers preferably be configured to possess such sufficient thicknesses as described above. In this instance, by holding the concentrations of ascorbic acid added to the coating layer Cm* sufficiently high, it is possible to substantially prevent the decrease in the sustained release rate of ascorbic acid.

And it is also possible to keep the sustained release rate from decreasing significantly by adding some water-soluble polymer to the coating layers with relative thicker structure.

Considering all factors described above, generally, the coating layer thickness Ld is to be formed basically to be 50 nm or more and 7000 nm or less, preferably 100 nm or more and 5000 nm or less, more preferably 1000 nm or more and 3000 nm or less. And within this range in layer thickness, it is required to take into considerations the several inner body conditions in which the anti-adhesion material is applied, such as inner pressure of abdominal cavity, environment of inner force, type of organs to be applied, and required duration time for sustained releases, and on these factors, it is decide which is given more priority with regard to sustained-releasability or shape-holding capability, and thus, the thickness of coating layer (and added ascorbic acid concentration Cm* in it) to be adopted is preferably determined. (However, in any case, these two factors: stained-releasability or shape-holding capability must be considered.)

### Examples

### [Examples (A)]

Based on the configuration of the anti-adhesion material 1 according to the typical embodiment as shown in Fig. 1, samples of the working examples (afterward may be referred to as just "examples") were prepared by adding a predetermined amount of L-ascorbic acid (manufactured by Wako Pure Chemical Industries) to a material forming a base body layer, as described later, by arranging a first coating layer and a second coating layer (hereinafter sometimes abbreviated simply as "coating layers") made of the same material on both surfaces of the base body layer formed into a film shape, and by making an adjustment of pH level into a predetermined range as described above (samples of comparison examples were also prepared without adding L-ascorbic acid).

### (Formation of Base body Layer)

Specifically, first, a water-soluble polymer, pullulan, was used as the material of the base body layer with predetermined amount of the ascorbic acid added thereto, and a film made of pullulan of 100 mm×120 mm×50 µm in thickness was formed by a flow casting method so as to form an ascorbic acid containing base body layer. (ascorbic acid concentration in base body layer: Cm)

### (Formation of Coating Layers)

Subsequently, the coating layers were formed by a conventional dipping method. In particular, after preparing a toluene solution of poly(aliphatic ester), i.e., polylactic acid-polyglycolic acid-poly ε-caprolactone, adjusted to a predetermined concentration (hereinafter referred to as a coating solution), the prepared base body layer was dipped into the coating solution to form coating layers made of a polylactic acid-polyglycolic acid-poly ε-caprolactone terpolymer on both surfaces of the base body layer. After the dipping, the layers were dried at room temperature for 30 minutes to 1 hour to acquire samples (test pieces) for the examples.

### [Comparative Examples]

Anti-adhesion materials were fabricated in the same way as the working examples except that no ascorbic acid was added to (or contained in) the base body layer of the examples so as to acquire samples (test pieces) for the comparison examples.

### (Method of Measuring Optical Thickness of Coating Layers)

The coating layers of the present invention are extremely thin films and are therefore preferably indicated by optical thickness (Ld).

The optical thickness (Ld) of the coating layers laminated on the base body layer was measured by using a spectroscopic ellipsometer ("alpha-SE (registered trademark in the U.S.A.)" manufactured by J. A. Woollam Japan). The measurement wavelength was 380 nm to 900 nm.

### (Examples 1 to 3) (Study on Additive Concentration of Ascorbic Acid Cm*)

The thickness (Ld) of the coating layers made of polylactic acid-polyglycolic acid-poly ε-caprolactone described above was fixed to 300 nm and the added ascorbic acid concentration Cm (w/w)% in the base body layer was varied, and pH (24) values after 24 hours from dipping into bovine plasma were measured. The results are described in Table 1. Specifically, Example 1 resulted in pH (24) = 7.0 at the added ascorbic acid concentration Cm = 2.0 (w/w)%; Example 2 resulted in pH (24)=7.0 at Cm = 4.0 (w/w) %; and Example 3 resulted in pH (24) =6.0 at Cm = 8.0 (w/w) %.

### (Comparative Example 1) (Ascorbic Acid Not Added)

For Comparative Example 1, the same test as Examples 1 to 3 (the coating layer thickness (Ld) = 300 nm) was conducted except for the absence of ascorbic acid (Cm = 0 %) in the base body layer. The pH (24) after 24 hours from dipping into bovine plasma was 7.3. The result was shown in Table 1.

### (Example 4) (Study on Coating Layer Thickness (Ld))

The same test as Example 3 was conducted except that the coating layer thickness (Ld) was set to 75 nm. Consequently, as described in Table 2, in the case of Cm = 8.0 %, no difference was recognized from pH (24) = 6.0 in the case of Ld = 300 nm.

### (Comparative examples 2 to 3) (Study on Coating Layer Thickness (Ld))

The same test as Comparative Example 1 was conducted except that the coating layer thickness (Ld) was set to 75 nm (Comparison Example 2) or 600 nm (Comparison Example 3). The result was shown in Table 2 along with Comparison Example 1 (Ld = 30 nm). Comparative Examples without addition of ascorbic acid (Cm = 0 %) in the base body layer resulted in pH(24) = 7.3 (constant) even when the coating layer thickness (Ld) was changed to 75 nm→300 nm→600 nm.

### (Ascorbic Acid Additive Amount and pH)

**[Table 1]**

| | Comparative Example 1 | Example 1 | Example 2 | Example 3 |
|---|---|---|---|---|
| coating layer optical thickness Ld (nm) | 300 nm | 300 nm | 300 nm | 300 nm |
| ascorbic acid concentration Cm (w/w) (%) | 0 % | 2.0 % | 4.0 % | 8.0 % |
| pH (24 hr) after dipping in bovine plasma | 7.3 | 7.0 | 7.0 | 6.0 |

### (Coating Layer Optical Thickness and Ascorbic Acid Additive Amount)

**[Table 2]**

| | Comparative Example 2 | Comparative Example 1 | Comparative Example 3 | Example 4 |
|---|---|---|---|---|
| coating layer optical thickness Ld (nm) | 75 nm | 300 nm | 600 nm | 75 nm |
| ascorbic acid concentration Cm (w/w) (%) | 0 % | 0 % | 0 % | 8.0 % |
| pH (24 hr) after dipping in bovine plasma | 7.3 | 7.3 | 7.3 | 6.0 |

### [Test Example I] (Evaluation of Anti-adhesion Performance)

Test Example I is a test for evaluating an anti-adhesion performance of an anti-adhesion material (Test Example 1 corresponds to Test Example 2 of Patent Document 1 of the applicants).

To evaluate the anti-adhesion performance, test pieces were prepared from the sampled related to examples and comparative examples and the test pieces were applied inside the abdominal cavity of a pig to observe and score a degree of adhesion in order to evaluate the anti-adhesion performance.

### (1) Preparation of Test Pieces

The samples obtained in Examples 1 to 4 and Comparative examples 1 to 3 described above were cut to prepare 100mm×120 mm rectangular test pieces.

### (2) Test Method (Production of Adhesion Model)

First, the abdominal cavity of a pig was opened by 15-cm median incision of the abdomen under general anesthesia and the small intestine was externally exposed. A chosen constant area (about 1×5 cm) of the exposed small intestine was abraded by using a file until petechial hemorrhage occurred. After the petechial hemorrhage occurred, the area was exposed into air exactly for 10 minutes. The small intestine was returned into the abdominal cavity, and the anti-adhesion material according to each of the test pieces described above was applied directly just below the portion of the incision. The abdominal wall was continuously sutured in two layers by using absorbable sutures (2-0) and was closed to produce an adhesion model. The number of examples was approx. 5 for each group in both the examples and the comparative examples.

### (3) Test Method (Observation of Degree of Adhesion, Calculation of Adhesion Score)

After 14 days from the production of the adhesion model, the pig was exsanguinated and sacrificed under general anesthesia, and the abdominal cavity was opened to observe the adhesion directly below the median wound with the naked eye so as to obtain a probability of occurrence of adhesion. For the purpose of evaluation of the lower abdomen in this test, the adhesion of the liver in the upper abdomen was ignored.

The test result was shown in Tables 3 and 4. Regarding descriptions of Tables 3 and 4, for example, the field of "Example 1" of Table 3 (the coating layer thickness (Ld) 300 nm, the ascorbic acid addition concentration Cm = 2.0 %) corresponds to "adhesion occurrence rate σ: 20 % (1/5)" of Table 4 (the field of the coating layer thickness (Ld) 300 nm, the ascorbic acid addition concentration Cm = 2.0 (w/w)%), and the field of "Comparative Example 2"of Table 3 (the coating layer thickness (Ld) 75 nm, the ascorbic acid addition concentration Cm = 0 %)corresponds to "adhesion occurrence rate σ: 75 % (3/4) " of Table 4 (the field of the coating layer thickness (Ld) 75 nm, the ascorbic acid addition concentration Cm = 0 %).

**[Table 3]**

| ascorbic acid concentration Cm (w/w)(%) | coating layer optical thickness Ld (nm) | | |
|---|---|---|---|
| | 75 nm | 300 nm | 600 m |
| 0% | Comparative Example 2 | Comparative Example 1 | Comparative Example 3 |
| 2.0% | | Example 1 | |
| 4.0% | | Example 2 | |
| 8.0% | Example 4 | Example 3 | |

**[Table 4]**

| adhesion occurrence rate σ other than liver (number of pigs with adhesion/the actual number of pigs) | coating layer optical thickness Ld (nm) | | |
|---|---|---|---|
| | 75 nm | 300 nm | 600 nm |
| Cm = 0% | 75% (3/4) | 66% (2/3) | 50% (2/4) |
| Cm = 2.0% | | 20% (1/5) | |
| Cm = 4.0% | | 0% (0/5) | |
| Cm = 8.0% | 50% (3/6) | 0% (0/5) | |

### (Discussion 1)

### (1) (Study on Ascorbic Acid Addition Concentration (i))

From Table 4, comparing the results of Example 1 (the adhesion occurrence rate σ: 20%) and Examples 2, 3 (the adhesion occurrence rate σ: 0%) in which a change in ascorbic acid in the base body layer from the predetermined addition concentration Cm (2.0 (w/w)% to 8.0 (w/w)%) was made at the coating layer thickness (Ld) set to 300 nm with the Comparison Example 1 (the adhesion occurrence rate σ: 66 %) without addition of ascorbic acid at the same coating layer thickness (Ld) of 300 nm, a noticeable reduction in the adhesion occurrence rate was confirmed in the case of Examples. Comparing Example 1 and Examples 2, 3, it was confirmed that when the ascorbic acid addition concentration Cm is increased from 2.0 (w/w) % to 4.0-8.0 (w/w) %, the adhesion occurrence rate σ was further reduced from 20 % to 0 %.

### (2) (Study on Ascorbic Acid Addition Concentration (ii))

When the coating layer optical thickness (Ld) was fixed at 75 nm, it was confirmed that the adhesion occurrence rate o was reduced in Example 4 (the adhesion occurrence rate σ = 50%) with ascorbic acid added at high concentration (Cm = 8.0(w/w)%) in the base body layer as compared to Comparative Example 2 (the adhesion occurrence rate σ= 75%) without addition of ascorbic acid.

### [Test Example II]

Test Example II is a test for evaluating the sustained release of ascorbic acid for achieving the anti-adhesion performance. In particular, a comparison was made, at the same ascorbic acid addition concentration in the base body layer Cm = 8.0 w/w% (constant value) between examples different only in the coating layer thickness Ld ([Example 3] (Ld = 75 nm) and [Example4] (Ld = 300 nm)) to confirm whether the optimum sustained release was achieved.

### (1)Preparation of Test Pieces

The samples obtained in the examples and the comparison examples described above were cut to prepare 50 mm×50 mm square test pieces.

### (2) Test Method

The anti-adhesion materials of Example 3 (Ld = 75 nm) and Example 4 (Ld = 300 nm) each was immersed in 100 mL of a phosphate buffer solution (pH 7.4) to measure the ascorbic acid concentration in the phosphate buffer solution over the time for 24 hours . The ascorbic acid was quantified by using a vitamin C quantification kit (manufactured by Cosmo Bio) with a colorimetric determination method.

Assuming that the concentration was 100% when the whole amount of the ascorbic acid contained in the anti-adhesion material was eluted into a phosphate buffer solution, the sustained release rate (ϕ) was obtained at each measurement point. The materials used in the test were observed in terms of shape after 24 hours.

### (Test Results)

The test results are shown in Fig. 2. Data was not collected after 24 hour since the ascorbic acid was degraded.

In the data within 24 hours, Example 3 showed the sustained release rate ϕ of 60 to 70 % and Example 4 showed the sustained release rate ϕ of about 20 to 30 %. Comparing both examples, Example 4 (Ld = 75 nm) having only the thinner coating layer thickness Ld had a sustained release rate much larger than the Example 3 (Ld = 3 00 nm) having the larger coating layer thickness, resulting in elution of 70% or more at the elapsed time T = 10 hr. In contrast, it is demonstrated that the sustained release was much slower in Example 4 (Ld = 300 nm) with the thicker coating layer (ϕ = about 30% even at T = 24 hr).

However, with regard to the adhesion occurrence rate σ after T=14 days, Example 3 showed the adhesion occurrence rate σ of 0 % and produced the anti-adhesion effect far more excellent than Example 4, which showed σ of 50%. It is presumed that this is because the optimum sustained release rate was maintained for T = 14 days in Example 3. Conversely, in the case of Example 4, it is estimated that the ascorbic acid in the base body layer was depleted in a short time because the sustained release rate was too large.

The materials (test pieces) used in the test were almost gelled after 24 hours and was able to be considered as semisolids although the shape was maintained.

### (Discussion 2)

It was found that the sustained release rate varies when a solid or semisolid material is used. More specifically, by disposing a coating layer made of a biodegradable poly (aliphatic ester) and by controlling the thickness thereof in a range of 75 nm or more and less than 600 nm, the sustained release of ascorbic acid (added to the base body layer) can be maintained for a long time. This means that the usable material characteristics can be adjusted in accordance with a site or a depth of damage to be accommodated or applied. It is also shown that the sustained release does not occur instantaneously (in whole amount at one time) as in the case of a liquid material (e.g., Patent Document 2) and that the anti-adhesion material having the form of the base body layer of the present invention was capable of maintaining the sustained release over time (for a long period, e.g., 14 days as shown in the examples).

### [Examples (B)]

In the same manner as Examples (A), based on the configuration of the anti-adhesion material 1 according to the embodiment as shown in Fig. 1, samples of the working examples (afterward may be referred to as just "examples") were prepared by adding a predetermined amount of L-ascorbic acid (manufactured by Wako Pure Chemical Industries) to a material which was to be formed into a film-shaped base body layer. On both surfaces of the base body layer, a first coating layer on one surface and a second coating layer on the other (sometimes abbreviated simply as "coating layers") were formed, to which layers L-ascorbic acid was also added (samples of comparison examples were also prepared without adding L-ascorbic acid to both base body layer and coating layers).

### (Examples 5 to 6) (Formation of Ascorbic Acid Containing Relatively Thick Coating Layer)

### (Formation of Base Body Layer)

In the same manner as Examples (A), a water-soluble polymer, pullulan, was used as the material of the base body layer with predetermined amount (or Cm = 4 (w/w) %) of the ascorbic acid added thereto, and a film made of pullulan of 100 mm×120 mm×50 µm in thickness (L) was formed by a flow casting method so as to form an ascorbic acid containing base body layer.

### (Formation of Coating Layers)

Subsequently, the coating layers were formed by a dipping method. In particular, after preparing a toluene solution of poly(aliphatic ester), i.e., polylactic acid-polyglycolic acid-poly ε-caprolactone, adjusted to a predetermined concentration of poly(aliphatic ester) (hereinafter referred to as a coating solution), predetermined amount of the ascorbic acid (or Cm* = 0.4 (w/w) %) was added thereto. Into thus-prepared ascorbic acid containing coating solution (Cm* = 0.4(w/w)%), above-mentioned base body layer was dipped to form coating layers made of a polylactic acid-polyglycolic acid-poly ε-caprolactoneter polymer on both surfaces of the base body layer. After the dipping, the layers were dried at room temperature for 30 minutes to 1 hour. These coating (dipping) procedure and drying procedure were repeated to form coating layers (or first outer layer and second outer layer on both surfaces of the base body layer), (layer's ascorbic acid concentration Cm* =0.4 (w/w) %), having thicker layer thickness (Ld) compared with that of Examples 1-4. In these Examples, coating layer thickness Ld = 1000nm (Example 5), Ld = 3000nm (Example 6) were employed for samples (test pieces) for the experiments.

### (Comparative Example 4) (Ascorbic Acid Not Added)

For Comparative Example 4, in the same manner as Examples 5 to 6, samples (test pieces) for the experiments were prepared wherein to both surfaces of the base body layer (LD = 50pm), coating layers were formed (Ld = 1000 nm) except for the absence of ascorbic acid in the both layers (Cm = 0, Cm* = 0 %).

### [Test Example III] (Evaluation of Anti-adhesion Performance)

Test Example III, likewise as Test Example I, was for evaluating an anti-adhesion performance of an anti-adhesion material, wherein however, the study animal species was changed to one more akin to human, having an inner environment to which the anti-adhesion material must be applied, becomes severer owing to the presence of higher inner pressure of abdominal cavity or frequent inner organ movement etc. and the purpose of the test is to make sure whether the anti-adhesion material is applicable to this harsh environmental conditions.

To evaluate the anti-adhesion performance, test pieces were prepared from the samples related to Examples (B) and comparative examples and the test pieces were applied inside the abdominal cavity of a monkey (Macaca fascicularis) to observe the degree of adhesion and by measuring the size (length) of developed adhesion, evaluation of the anti-adhesion performance was made.

### (1) Preparation of Test Pieces

The samples obtained in Examples 5 to 6 and Comparative example 4 described above were cut to prepare 100mm×120 mm rectangular test pieces.

### (2) Test Method (Production of Adhesion Model)

First, the abdominal cavity of a monkey was opened by 8-cm median incision of the abdomen under general anesthesia and the large intestine was externally exposed.

A chosen constant area (about 3×4 cm) of the exposed large intestine was abraded by using a file until petechial hemorrhage occurred.

After the petechial hemorrhage occurred, the area was exposed into air exactly for 10 minutes. The large intestine was returned into the abdominal cavity, and the anti-adhesion material according to each of the test pieces described above was applied directly just below the portion of the incision. The peritoneal membrane, abdominal wall, and muscular layer were continuously sutured by using nylon sutures (3-0), and subcutaneous and skin were simply ligated using nylon sutures (4-0) and closed to produce an adhesion model. The number of examples was approx. 3 for each group in both the examples 5-6 and the comparative example 4.

### (3) Test Method (Observation of Degree of Adhesion, Measurement of Rate of Adhesion Length)

After 14 days from the preparation of the adhesion model, the monkey was exsanguinated under general anesthesia, and the abdominal cavity was opened to observe the adhesion directly below the median wound with the naked eye so as to obtain a probability of occurrence of adhesion and calculate a rate of adhesion length.

### (Discussion 3)

In Examples 5 to 6, on both surfaces of the base body layer having same thickness as Examples 1 to 4 (LD = 50µm, composed of pullulan), rather thicker coating layers of poly(aliphatic ester)were formed in an attempt to enhance the mechanical strength of the anti-adhesion material and thereby reliably maintain its shape intact while being placed in the living body. Such thicker coating layers were first expected to have the possibility to prevent sustained release of ascorbic acid from the base body layer. However, in that case (Ld = 1000 nm (Example 5), Ld = 3000 nm (Example 6)), it was found that the adhesion occurrence rate σ could be substantially reduced by adding and incorporating ascorbic acid into the thick coating layers as shown in Table 5.

(In addition, it was confirmed that the shape of applied anti-adhesion material maintained almost its initial shape after 14 days, showing that the anti-adhesion material could achieve its primary objective with regard to shape-sustainability by providing the anti-adhesion material with such thick coating layer as used in the Examples 5 to 6.)

Specifically, as shown in Table 5, in Example 5, wherein ascorbic acid concentration in the base body layer Cm = was 4.0 (w/w) % and the coating layer thickness (Ld) was set to 1000 nm, and even for such a thick coating layer constitution, by maintaining its ascorbic acid concentration (Cm* = 0.4 (w/w) %), it was shown that adhesion occurrence rate σ was reduce to 67% (2/3) and adhesion length rate *β* (= I*/I) was lowered to 15.5%.

This result of Example 5 was evaluated as a remarkable improvement in the anti-adhesion capability compared to that of Comparative Example 4, in which the coating layer thickness (Ld) was same (= 1000 nm) and no ascorbic acid was added to both the base body layer and coating layer (Concentration Cm = 0%, Cm* = 0%),with the result that adhesion occurrence rate σ was 100% (3/3) and adhesion length rate *β* (= I*/I) was 82.60%.

Meanwhile, in Example 6, the coating layer thickness (Ld) was further increased to 3000 nm. Even in such an extreme case, as well as Example 5, by keeping the levels of ascorbic acid concentration in the base body layer Cm = was 4.0 (w/w)% and that of in the coating layer Cm*= 0.4 (w/w)%), adhesion occurrence rate σ was reduced to 33% (1/3) and adhesion length rate *β* (= I*/I) was lowered to 33.30%.

Further, comparison of Example 2 (Table 3-4) and Examples 5-6 (Table 5) was made and some difference was noticed. Specifically, in Example 2, wherein adhesion occurrence rate σ was 0 (zero) (no adhesion was occurred) (Table 4) for the layer configuration of base body (LD = 50µm, ascorbic acid concentration Cm = 4.0(w/w)%) and coating layer (Ld = 300nm, ascorbic acid concentration Cm* = 0 (zero)(w/w)%). While, in Examples 5-6, adhesion occurrence rate σ was not completely reduced to zero. The primary reason for this apparent difference is fundamentally considered to stem from the difference in studied animal species (swine versus monkey) which inherently caused different basic frequencies of adhesion at the base line. And also, part of the reason for the difference is that in Examples 5-6, to maintain the mechanical strength of anti-adhesion material, far thicker coating layers (Ld = 1000 nm of 3000 nm) were provided. However, the difference between the two Examples were not considered to be of substantial, or not derived from the inherent or actual capability of the studied anti-adhesion materials.

One of the measures to address this difference (or to bridge this difference gap) is to gradually increase the amount of ascorbic acid Cm* to be added to the coating layer in the manner like, 0.4% → 0.6% → 0.8% → 1.0%, adhesion occurrence rate σ would be expected to be substantially lowered. It would also be expected that by adopting some measures such as an addition of small amount of water-soluble polymer (A), commensurate with ascorbic acid, in the coating layer Ld (which is mainly consisted of biodegradable poly(aliphatic ester)(B)), would further accelerate the sustained release of ascorbic acid and thus furthermore reduce the adhesion occurrence rate o.

**[Table 5]**

| | Coating Layer thickness Ld (nm) | Added Ascorbic Acid Concentration Cm, Cm* (w/w)(%) | | Adhesion Occurrence Rate σ (Number of Adhered Monkeys/ Actual Number of Monkeys) | Rate of Adhesion Length β(= I*/I) (Length of Adhered Incisional Wound/Length of Incisional Wound) |
|---|---|---|---|---|---|
| | | Base Layer Concentra tion Cm | Coating Layer Concentra tion Cm* | | |
| Comparative Example 4 | 1000 | 0% | 0% | 100%(3/3) | 82.60% |
| Example 5 | 1000 | 4.0% | 0.4% | 67%(2/3) | 15.50% |
| Example 6 | 3000 | 4.0% | 0.4% | 33%(1/3) | 33.30% |

Although tests corresponding to Test Example 1, Test Example 3, and Test Example 4 described in Patent Document 1 were conducted for confirmation, details will not be described here since it was found that no functional or actual problem exists in practical use as described below.

### (i) (Test Example 1) (Evaluation of Degradability When Anti-adhesion Material Is Applied to Living Tissue)

Although a time until development of strength after applying to a living body becomes slightly longer, the function is not affected.

### (ii) (Test Example 3) (Evaluation of Handleability When Anti-adhesion Material Is Wetted)

When applied inside the abdominal cavity of a swine or monkey in Test Example 2, the material was able to be applied without any problems or difficulty.

### (iii) (Test Example 4) (Evaluation of Contact Properties When Anti-adhesion Material Is Applied to Living Tissue)

When applied inside the abdominal cavity of a swine or monkey in Test Example 2, the material was able to be applied without any problems or difficulty.

### INDUSTRIAL APPLICABILITY

Since the anti-adhesion material of the present invention basically has a configuration comprising an ascorbic acid or an ascorbic acid derivative contained as an antioxidant in a solid or semisolid base body made of a water-soluble polymer (A) and a biodegradable poly (aliphatic ester) (B), the base body made of a water-soluble polymer and a poly (aliphatic ester) acts as a physical barrier inserted between surfaces of wound and the healing on the wound surfaces is promoted by the ascorbic acid or the derivative thereof being sustained-released, so that the anti-adhesion function is extremely efficiently achieved in a synergistic manner, and therefore, the industrial availability in the medical fields extremely high.

### EXPLANATIONS OF LETTERS OR NUMERALS

1 anti-adhesion material
10 base body layer
20 first coating layer (first outer layer)
30 second coating layer (second outer layer)
Cm Concentration of ascorbic acid etc. in the base layer
Cm* Concentration of ascorbic acid etc. in the coating layer (outer layer)
LD thickness of base layer
Ld thickness of coating layer (optical thickness)
I length of incision wound
I* length of adhesion of incision wound
ϕ sustained release rate of ascorbic acid (elusion rate)

## Claims

1. An anti-adhesion material comprising an ascorbic acid or an ascorbic acid derivative contained as an antioxidant in a solid or semisolid base body made of a water-soluble polymer and a poly(aliphatic ester).

2. The anti-adhesion material according to claim 1, wherein
the solid base body has any one form selected from a film shape, a sheet shape, and a mesh shape, wherein
the forms of the film shape, the sheet shape, and the mesh shape have a single layer structure substantially constituted by the base body or a laminated structure including a coating layer made of a poly(aliphatic ester) on a base body layer of the base body, and wherein
the form of the mesh shape has a fibrous structure made up of a composite body acquired by forming a solid base body made of a water-soluble polymer and a poly (aliphatic ester) into a rod shape and has a weight of 0.8 g/m² to 830 g/m².

3. The anti-adhesion material according to claim 1, wherein the semisolid base body has a gel form including a colloidal form with a single layer structure or a laminated structure and has a viscosity of 100 Pa·s to 1,000,000 Pa·s at 37 °C.

4. The anti-adhesion material according to claim 2, wherein the laminated structure including a coating layer on the base body layer of the base body includes respective coating layers on both surfaces of the base body layer and is formed in three layers, comprising the coating layers on both surfaces and the base body layer.

5. The anti-adhesion material according to claim 4, wherein the thickness of the coating layers is 50 nm or more and less than 7000 nm in optical thickness of the coating layers when measured by using a spectroscopic ellipsometer at a wavelength of 380 nm to 900 nm.

6. The anti-adhesion material according to claim 1, wherein the ascorbic acid or the derivative thereof is contained at 0.1 (w/w) % or more to 20 (w/w) % or less relative to a weight of a base body material constituting the base body or the base body layer.

7. The anti-adhesion material according to any of claims 1 to 6, wherein when the anti-adhesion material is dipped in bovine plasma, pH becomes 7.5 to 6.0 after 24 hours.

8. The anti-adhesion material according to any one of claims 2 to 7, wherein
The anti-adhesion material includes as the coating layers a first coating layer disposed on one surface of the base body layer and a second coating layer disposed on the other surface of the base body layer, and wherein the first coating layer and the second coating layer are made of the same material.

9. The anti-adhesion material according to any one of claims 2 to 7, wherein
The anti-adhesion material includes as the coating layers a first coating layer disposed on one surface of the base body layer and a second coating layer disposed on the other surface of the base body layer, and wherein the first coating layer and the second coating layer are made of different materials.

10. The anti-adhesion material according to any one of claims 1 to 9, wherein
the water-soluble polymer is a polysaccharide, a protein, or a synthetic polymer.

11. The anti-adhesion material according to any one of claims 1 to 10, wherein the water-soluble polymer (A) and the poly(aliphatic ester) (B) constituting the base body have a weight proportion ω (= A/B) of 1 to 99/99 to 1.

12. An anti-adhesion material comprising an ascorbic acid or an ascorbic acid derivative contained as an antioxidant in a solid or semisolid base body substantially made of a water-soluble polymer, the anti-adhesion material further comprising respective coating layers made of a poly(aliphatic ester) on both surfaces of a base body layer of the base body.

13. The anti-adhesion material according to any one of claims 2 to 12, wherein the anti-adhesion material comprises a plant-derived antioxidant including carotenoids that are plant lipophilic pigments or polyphenols contained as the antioxidant of claim 1.
